# EUROPÄISCHE PATENTANMELDUNG

(11) **EP 2 561 853 A1**
(43) Veröffentlichungstag der Anmeldung: **27.02.2013**
(21) Anmeldenummer: 11178665.3
(22) Anmeldetag: 24.08.2011
(51) Int. Cl.: A61K 8/04, A61K 8/37, A61K 8/49, A61K 8/92, A61Q 17/04

(54) **Sonnenschutzspray mit Sonnenschutzfaktor SPF 20 bis SPF 30**

(71) Anmelder: PM-International AG, 1618 Luxembourg (LU)
(72) Erfinder: Golz-Berner, Karin, 98000 Monaco (MC); Sorg, Rolf, L-5444 Schengen (LU); Meinhard, Horst, D-56249 Herschbach (DE)
(74) Vertreter: Gulde Hengelhaupt Ziebig & Schneider

(57) **Zusammenfassung**

Die Erfindung betrifft einen Sonnenschutzspray mit einem hohen Sonnenschutzfaktor von SPF 20 bis SPF 30, der wasserfrei und transparent ist. Der Sonnenschutzspray umfaßt eine Kombination der kosmetischen UV-Filter Ethylhexyl Methoxycinnamate, Ethylhexyl Triazone und Bis-Ethylhexyloxyphenol Methoxyphenyl Triazine und eine Kombination der kosmetischen Öle und Ester C12-15 Alkyl Benzoate, Dicaprylyl Carbonate, Meadowfoam Oil, Propylheptyl Caprylate, Isoamyl Cocoate, Phenoxyethyl Caprylate, und Diisopropyl Sebacate. Zusammen mit weiteren kosmetischen Hilfsstoffen stellt der Spray eine transparente, wasserfreie Lösung dar.

## Beschreibung

Die Erfindung betrifft einen Sonnenschutzspray mit einem hohen Sonnenschutzfaktor von SPF 20 bis SPF 30, der wasserfrei und transparent ist.

Aus dem Stand der Technik sind eine Vielzahl von Sonnenschutzformulierungen bekannt, die jedoch regelmäßig Emulsionen vom Typ W/O oder OIW darstellen, da sehr viele Bestandteile in solchen Formulierungen wasserlöslich sind. Darüber hinaus besteht bei Sprays das Problem, daß eine sehr niedrige Viskosität eingestellt werden muß und eventuelle ungelöste Fettstoffe die Düsenöffnungen des Applikators passieren müssen. Eine solche Emulsion mit SPF 20-25 ist z.B. aus der KR 2010 0039955 bekannt. Ebenfalls ein Sonnenschutzspray auf Emulsionsbasis mit einem SPF-Booster und dispergierbaren teilchenförmigen Bestandteilen ist aus der US 2007/178057 bekannt.

Der Erfindung liegt die Aufgabe zugrunde, einen Sonnenschutzspray mit einem hohen Sonnenschutzfaktor von SPF 20 bis 30 bereitzustellen, bei dem die UV-Filter vollständig in gelöster Form vorliegen und bei dem kein Wasserzusatz erfolgt.

Eine weitere Aufgabe besteht in der Bereitstellung eines Sonnenschutzsprays mit hohem Sonnenschutzfaktor, bei dem der Sonnenschutzfaktor über einen breiten Temperaturbereich von 4 bis 50°C im wesentlichen konstant bleibt.

Eine weitere Aufgabe besteht in der Bereitstellung eines Sonnenschutzsprays mit hohem Sonnenschutzfaktor, bei dem kein Auskristallisieren von UV-Filtern bei Temperaturen ab 12°C erfolgen kann

Eine weitere Aufgabe besteht darin, einen Sonnenschutzspray bereitzustellen, bei dessen Benutzung durch den Anwender in dessen Kleidung Flockenbildung auf den Textilien beim Schwitzen an den entsprechenden Kontaktstellen zwischen Haut und heller Kleidung vermieden wird.

Eine weitere Aufgabe besteht in der Angabe eines Verfahrens zur Behandlung der Haut mit diesem Sonnenschutzspray.

Erfindungsgemäß umfaßt der Sonnenschutzspray eine Kombination der kosmetischen UV-Filter Ethylhexyl Methoxycinnamate, Ethylhexyl Triazone und Bis-Ethylhexyloxyphenol Methoxyphenyl Triazine; eine Kombination der kosmetischen Öle und Ester C12-15 Alkyl Benzoate, Dicaprylyl Carbonate, Meadowfoam Oil, Propylheptyl Caprylate, Isoamyl Cocoate, Phenoxyethyl Caprylate, und Diisopropyl Sebacate und stellt zusammen mit weiteren kosmetischen Hilfsstoffen eine transparente, wasserfreie Lösung dar.

Es wurde gefunden, daß durch eine bestimmte Auswahl von UV-Filtern und speziellen Ölen und Estern sowohl der hohe Sonnenschutzfaktor von SPF 20 bis 30 als auch die Beständigkeit des Lösungsverhaltens der UV-Filter bei gleichzeitiger Erhaltung einer transparenten Lösung gewährleistet ist.

Insbesondere treten keine Kristallisationserscheinungen der UV-Filter bei tiefen Temperaturen von unter 10°C auf, so daß der Sonnenschutzspray auch im Winter nutzbar ist unter Erhaltung des vorgegebenen Sonnenschutzfaktors. Bei bekannten Sonnenschutzemulsionen mit hohem Lichtschutzfaktor treten bei niedrigen Temperaturen stets Verluste des Lichtschutzfaktors auf, meist infolge von Kristallisationserscheinungen der UV-Filter.

Ein weiterer Vorteil des erfindungsgemäßen Sonnenschutzsprays ist die Textilechtheit seiner Bestandteile, d.h., es gibt keine Fleckenbildung in hellen Textilien durch Schweiß und Spraybestandteile. Weiterhin gibt es keine Klebrigkeitserscheinungen des Sprays nach dem Aufsprühen auf die Haut nach Ablauf einer kurzen Trocknungsphase.

Ein weiterer Vorteil der Erfindung besteht in einem ausgewogenen Schutz vor UV-A-und UV-B-Strahlen.

Vorzugsweise liegt der Anteil aller kosmetischen UV-Filter im Bereich von 5 bis 20 Gew.-%, wobei die Prozentangaben auf das Gesamtgewicht des Sonnenschutzsprays bezogen sind.

Dabei liegt der Anteil von Ethylhexyl Methoxycinnamate im Bereich von 4 - 10 Gew.-%, von Ethylhexyl Triazone im Bereich von 0,5 bis 3 Gew.-% und von Bis-Ethylhexyloxyphenol Methoxyphenyl Triazone im Bereich von 0.5 bis 6 Gew.-%, jeweils bezogen auf das Gesamtgewicht des Sprays.

Vorzugsweise liegt der Artfieil aller kosmetischen Öle und Ester im Bereich von 20 bis 40 Gew.-%

Dabei liegt der Anteil der Öle und Ester vorteilhaft in folgenden Bereichen (in Gew.-%): Meadowfoam Oil 1 - 2 %, C12-15 Alkylbenzoate 20-25 %, Dicaprylyl Carbonate 3-8 %, Propylheptyl Caprylate 1-1,5 %, Isoamyl Cocoate Phenoxyethyl Caprylate 1-2 %, jeweils bezogen auf das Gesamtgewicht des Sprays.

Als Lösungsmittel für alle Bestandteile wird Ethanol eingesetzt.

Der erfindungsgemäße Sonnenschutzspray kann weitere kosmetische Hilfsstoffe enthalten, die u. a. auch unter dem Gesichtspunkt der Transparenz des Sprays auszuwählen sind. Dazu gehören weitere Öle, Ester, Copolymere, Antioxidationsmittel, Radikalfänger und Gemische davon.

Besonders vorteilhaft sind Canoia Oil, Cyclopentasiloxane, Dimethicone, Tocopheryl Acetate, Acrylates / Octylacrylamide Copolymer, Ascorbyl Palmitate, NTC-Komplex (von PM-International, Luxemburg), Coffea Arabica Seed Oif, Phytosolve® 9940 (von Lipoid GmbH, Ludwigshafen), Squalane / Carotinoid, Photosome, Vitamin E und Gemische davon.

Der NTC-Komplex besteht aus farblosen öllöslichen Carotinoiden (EP 1107723) und einer wasserlöslichen Aminosäure (Thiotamine) in nanomizellierter Form.

Phytosolve 9940® ist ein Produkt auf Basis von Sojalecithin.

Das Gemisch von Isoamyl Cocoate und Phenoxyethyl Caprylate liegt vorteilhaft im Verhältnis 1:1 bis 1:2 vor.

Der erfindungsgemäße Sonnenschutzspray ist wasserfrei. Das bedeutet, daß kein Wasser der Mischung zugesetzt wird. Eventuelle Restanteile von Wasser in den zugesetzten Rohstoffen sind denkbar, sollten jedoch unter 0.01 % liegen, bezogen auf das Gesamtgewicht des Sprays.

Ein besonders vorteilhafter Komplex von wirksamen Estern und kosmetischen Hilfsstoffen besteht aus Isoamyl Cocoate, Phenoxyethyl Caprylate, Vitamin E, NTC-Komplex und Squalane & Dunaliella Salina Extract, dessen Wirksamkeit das Lösungsvermögen für die UV-Filter noch mehr erhöht.

Ein bevorzugter Sonnenschutzspray für den Lichtschutzfaktor SPF 30 enthält die folgenden Bestandteile (in Gew.-%)

| | |
|---|---|
| C12-15 Alkyl Benzoate | 20 bis 25 |
| Dicaprylyl Carbonate | 3 bis 8 |
| Ethylhexyl Methoxycinnamate | 8 bis 10 |
| Ethylhexyl Triazone | 2 bis 3 |
| Bis-Ethylhexyloxyphenol Methoxyphenyl Triazine | 4 bis 6 |
| Meadowfoam Oil | 1 bis 2 |
| Canola Oil | 1 bis 3 |
| Diisopropyl Sebacate | 3 bis 7 |
| Cyclopentasiloxane | 8 bis 12 |
| Dimethicone | 0,1 bis 1,5 |
| Ascorbyl Palmitate | 0,05 bis 0,1 |
| Propylheptyl Caprylate | 1 bis 1,5 |
| Tocopheryl Acetate | 0,1 bis 0,2 |
| NTC-Komplex | 0,01 bis 0,02 |
| Ethanol | 30 bis q.s. |
| Acrylates / Octylacrylamide Copolymer | 0,3 bis 0,5 |
| Coffea Arabica Seed Oil | 0,05 bis 0,1 |
| Phytosolve® 9940 | 0,05 bis 0,15 |
| Squalane & Dunaliella Salina Extract | 0,05 bis 0,1 |
| Photosomes | 0,01 bis 0,1 |
| Isoamyl Cocoate, Phenoxyethyl Caprylate | 1 bis 2 |
| Vitamin E | 0,5 bis 1 |
| Parfümöl | 0,05 bis 0,2. |

Ein weiterer bevorzugter Sonnenschutzspray für den Lichtschutzfaktor SPF 20 enthält die folgenden Bestandteile (in Gew.-%)

| | |
|---|---|
| C12-15 Alkyl Benzoate | 20 bis 25 |
| Dicaprylyl Carbonate | 3 bis 8 |
| Ethylhexyl Methoxycinnamate | 5 bis 6 |
| Ethylhexyl Triazone | 1 bis 2 |
| Bis-Ethylhexyloxyphenol Methoxyphenyl Triazine | 0,5 bis 1,5 |
| Meadowfoam Oil | 1 bis 1,5 |
| Diisopropyl Sebacate | 5 bis 5,5 |
| Ascorbyl Palmitate | 0,05 bis 0,1 |
| Propylheptyl Caprylate | 1 bis 1,5 |
| Tocopheryl Acetate | 0,1 bis 0,15 |
| NTC-Komplex | 0,01 bis 0,02 |
| Ethanol | 30 bis q.s. |
| Acrylates / Octylacrylamide Copolymer | 0,3 bis 0,5 |
| Coffea Arabica Seed Oil | 0,05 bis 0,1 |
| Phytosolve® 9940 | 0,05 bis 0,9 |
| Squalane & Dunaliella Salina Extract | 0,05 bis 0,1 |
| Photosomes | 0,01 bis 0,1 |
| Isoamyl Cocoate, Phenoxyethyl Caprylate | 1 bis 2 |
| Vitamin E | 0,5 bis 1 |
| Parfümöl | 0,05 bis 0,2. |

Die Bestimmung des Sonnenschutzfaktors SPF erfolgt üblicherweise nach dem COLIPA-Verfahren (International Sun Protection Factor (SPF) Test Method - May 2006) (COLIPA - The European Cosmetic Toiletry and Perfumery Association - www.colipa.com). Eine weitere Methode, bei der speziell *in vivo*-UVA-Schutzfaktoren ermittelt werden, ist das Persistant Pigment Darkening-Verfahren (PPD) (Photodermatol. Photoimmunol. Photomed. 16, 245-249 (2000).

Die Erfindung betrifft weiterhin ein Verfahren zur Behandlung der Haut, bei dem man die Haut durch Entfernung fester Partikel reinigt, einen Sonnenschutzspray gemäß obiger Definition aufsprüht, den Spray 5 bis 10 Minuten trocknen läßt und gegebenenfalls einen zweiten dünnen Film aufsprüht und diesen in gleicher Weise trocknen läßt.

Durch das Aufsprühen auf die gereinigte Haut, die keine wesentlichen Feststoffpartikel wie Verschmutzungen oder Hautpartikelchen mehr aufweist, bildet sich ein gleichmäßiger und durchgehender Film mit den UV-Filtern auf der Haut aus, der gegebenenfalls durch eine Wiederholung des Sprühvorganges nach einer kurzen Trockenzeit von 5 bis 10 Minuten noch verstärkt werden kann. Bei einem eventuellen Hautkontakt mit Wasser (Dusche, Baden) reicht eine einmalige Wiederholung des Sprühvorganges, wenn zuvor bereits einmal gesprüht worden ist.

Die Erfindung wird nachstehend durch Beispiele näher erläutert. Die darin genannten Prozentangaben sind stets Gewichtsprozente.

### Beispiel 1 und 2 Sonnenschutzspray SPF 30

| Phase A | Bsp.1 | Bsp.2 |
|---|---|---|
| C12-15 Alkyl Benzoate | 20 bis 25 | 23 |
| Dicaprylyl Carbonate | 3 bis 8 | 3 |
| Ethylhexyl Methoxycinnamate | 8 bis 10 | 8,5 |
| Ethylhexyl Triazone | 2 bis 3 | 3,0 |
| Bis-Ethylhexyloxyphenol Methoxyphenyl Triazine | 4 bis 6 | 5,0 |
| Meadowfoam Oil | 1 bis 2 | 1,5 |
| Canola Oil | 1 bis 3 | 1,0 |
| Diisopropyl Sebacate | 3 bis 7 | 3,5 |
| Cyclopentasiloxane | 8 bis 12 | 8,5 |
| Dimethicone | 0,1 bis 1,5 | 1,0 |
| Ascorbyl Palmitate | 0,05 bis 0,1 | 0,05 |
| Propylheptyl Caprylate | 1 bis 1,5 | 1,2 |
| Tocopheryl Acetate | 0,1 bis 0,2 | 0,15 |

| Phase B | Bsp. 1 | Bsp. 2 |
|---|---|---|
| NTC-Komplex | 0,01 bis 0,02 | 0,01 |
| Ethanol | 30 bis q.s. | q.s. |
| Acrylates / Octylacrylamide Copolymer | 0,3 bis 0,5 | 0,35 |
| Coffea Arabica Seed Oil (Café Lipactif Raffine®) | 0,05 bis 0,1 | 0,05 |
| Phytosolve® 9940 | 0,05 bis 0,15 | 0,08 |
| Squalane & Dunaliella Salina Extract (IBR-CLC®) | 0,05 bis 0,1 | 0,06 |
| Photosomes | 0,01 bis 0,1 | 0,05 |
| Isoamyl Cocoate, Phenoxyethyl Caprylate | 1 bis 2 | 1,5 |
| Vitamin E | 0,05 bis 1 | 0,7 |
| Parfümöl | 0,05 bis 0,2 | 0,1 |

Die Phase A wird auf max. etwa 60°C erwärmt bis zum Erreichen einer homogenen Schmelze unter gleichzeitigem Rühren bei etwa 60 bis 100 U/min. Danach wird das erhaltene Gemisch auf 25 bis 28°C abgekühlt, wobei das Rühren fortgesetzt wird. Die Bestandteile der Phase B werden unter Rühren nacheinander zugegeben, wobei Schaumbildung vermieden wird. Danach wird vorsichtig homogenisiert. Man erhält eine klare Lösung mit einer Viskosität im Bereich von 1 bis 10 mPa·s, gemessen mit einem Brookfield-Viskosimeter bei 25°C mit Spindeln T-A, T-B.

### Beispiel 3 und 4 Sonnenschutzspray SPF 20

| Phase A | Bsp. 3 | Bsp. 4 |
|---|---|---|
| C12-15 Alkyl Benzoate | 20 bis 25 | 2,0 |
| Dicaprylyl Carbonate | 3 bis 8 | 5 |
| Ethylhexyl Methoxycinnamate | 5 bis 6 | 3 |
| Ethylhexyl Triazone | 1 bis 2 | 1,5 |
| Bis-Ethylhexyloxyphenol Methoxyphenyl Triazine | 0,5 bis 1,5 | 1,0 |
| Meadowfoam Oil | 1 bis 1,5 | 1,0 |
| Diisopropyl Sebacate | 5 bis 5,5 | 5,3 |
| Ascorbyl Palmitate | 0,05 bis 0,1 | 0,07 |
| Propylheptyl Caprylate | 1 bis 1,5 | 1,0 |
| Tocopheryl Acetate | 0,1 bis 0,15 | 0,1 |

| Phase B | Bsp. 3 | Bsp. 4 |
|---|---|---|
| NTC-Komplex | 0,01 bis 0,02 | 0,01 |
| Ethanol | 30 bis q.s. | q.s. |
| Acrylates / Octylacrylamide Copolymer | 0,3 bis 0,5 | 0,35 |
| Coffea Arabica Seed Oil | 0,05 bis 0,1 | 0,08 |
| Phytosolve® 9940 | 0,05 bis 0,9 | 0,7 |
| Squalane & Dunaliella Salina Extract | 0,05 bis 0,1 | 0,08 |
| Photosomes | 0,01 bis 0,1 | 0,04 |
| Isoamyl Cocoate, Phenoxyethyl Caprylate | 1 bis 2 | 1,5 |
| Vitamin E | 0,5 bis 1 | 0,5 |
| Parfümöl | 0,05 bis 0,2. | 0,1 |

Die Verfahrensweise zur Herstellung der transparenten Lösung entspricht der von Beispiel 1.

### Beispiel 5 Vergleichsversuch 1

Die Erhaltung des Sonnenschutzfaktors SPF 20 bei niedrigen Temperaturen wurde untersucht. Dazu wurde eine Zusammensetzung gemäß Beispiel 4 (Probe A) mit einem handelsüblichen Emulsionsspray SPF 20 (Probe B) bei 20°C und bei 10°C verglichen.

Die Sprays wurden auf die entsprechende Temperatur gekühlt und dort für wenigstens 2 h gehalten.

12 Testpersonen mit einer Haut vom Typ II/III mit mittlerer Bräunungsfähigkeit wurden nacheinander in einem auf 15°C temperierten Raum für 30 Minuten akklimatisiert. Die obere Seite des gewaschenen linken Unterarms der jeweiligen Testperson wurde durch dreimaliges Hin- und Herschwenken des Spraybehälters im Abstand von ca. 15 cm auf einer Fläche von ca. 60 cm² mit der Probe A dünn besprüht.

Nach einer Trockenzeit von 8 Minuten wurde der besprühte Hautbereich mit einem UV-Sonnensimulator (Xenon-Lampe) für 30 Minuten bestrahlt. Die gleiche Verfahrensweise erfolgte mit dem rechten Unterarm und mit Probe B.

Am Folgetag wurde die minimale erythemale Dosis (MED) visuell ermittelt und der SPF in üblicher Weise berechnet. Die Ergebnisse sind in Tabelle 1 enthalten.

**Tabelle 1**

| | SPF bei | | |
|---|---|---|---|
| | 20°C | | 10°C |
| Probe A | 19.6 | | 19,1 |
| Probe B | 19,4 | | 9,8 |

Aus dem geringerem SPF der Probe B bei 10 °C ist erkennbar, daß die Wirksamkeit der UV-Filter in einer Spray-Emulsion des Standes der Technik bei niedriger Temperatur deutlich nachläßt, was vermutlich mit Kristallisationserscheinungen zusammenhängt.

### Beispiel 6 Vergleichsversuch 2

Das Auftreten von Flecken in Textilien der Anwender wurde untersucht. Dazu wurde eine Zusammensetzung gemäß Beispiel 3 (Probe C) mit einem handelsüblichen Emulsionsspray SPF 30 (Probe D) verglichen.

12 Testpersonen mit einer Haut vom Typ II/III legten auf einem Kettler Sitz-Ergometer RX7 eine Trainingsstrecke von 10 km mit einer durchschnittlichen Geschwindigkeit von 22 km/h zurück. Die Pulsfrequenzobergrenze wurde mit 130/min festgelegt.

Vor dem Test wurde die frisch gewaschene und trockne Rückenfläche der jeweiligen Testperson durch mehrmaliges Hin- und Herschwenken des Spraybehälters im Abstand von ca. 15 cm auf einer Fläche von ca. 1400 cm² mit der Probe C (6 Personen) und mit der Probe D (6 Personen) dünn besprüht.

Nach einer Trockenzeit von 8 Minuten zogen die Testpersonen ein reinweißes Baumwoll-T-Shirt an und begannen mit dem Test.

Nach dem Test wurden die schweißfeuchten T-Shirts für 2 h trocknen gelassen bei ca. 25°C. Die visuelle Auswertung der T-Shirts der Träger mit der Probe C zeigte, daß diese nach wie vor reinweiß waren. Auf den T-Shirts der Träger mit der Probe D waren bei 4 Personen deutlich mehrere leicht gelbliche Flecken in unregelmäßiger Verteilung auf der Rückenfläche erkennbar. Die zeigt die klare Überlegenheit des erfindungsgemäßen Sprays C.

## Patentansprüche

1. Sonnenschutzspray mit Sonnenschutzfaktor SPF 20 bis SPF 30, **dadurch gekennzeichnet, daß** der Spray eine Kombination der kosmetischen UV-Filter Ethylhexyl Methoxycinnamate, Ethylhexyl Triazone und Bis-Ethylhexyloxyphenol Methoxyphenyl Triazine umfaßt;
und eine Kombination der kosmetischen Öle und Ester C12-15 Alkyl Benzoate, Dicaprylyl Carbonate, Meadowfoam Oil, Propylheptyl Caprylate, Isoamyl Cocoate, Phenoxyethyl Caprylate, und Diisopropyl Sebacate umfaßt
und der Spray zusammen mit weiteren kosmetischen Hilfsstoffen eine transparente, wasserfreie Lösung darstellt.

2. Sonnenschutzspray nach Anspruch 1, worin der Anteil aller kosmetischen UV-Filter im Bereich von 5 bis 20 Gew.-% liegt, wobei die Prozentangaben auf das Gesamtgewicht des Sonnenschutzsprays bezogen sind.

3. Sonnenschutzspray nach Anspruch 1 oder 2, worin der Anteil aller kosmetischen Öle und Ester im Bereich von 20 bis 40 Gew.-% liegt.

4. Sonnenschutzspray nach einem der Ansprüche 1 bis 3, worin das Lösungsmittel Ethanol ist.

5. Sonnenschutzspray nach einem der Ansprüche 1 bis 4, worin dieser weitere kosmetische Hilfsstoffe umfaßt, ausgewählt aus der Gruppe bestehend aus Canola Oil, Cyclopentasiloxane, Dimethicone, Tocopheryl Acetate, Acrylates / Octylacrylamide Copolymer, Ascorbyl Palmitate, NTC-Komplex, Coffea Arabica Seed Oil, Phytosolve 9940, Squalane & Dunaliella Salina Extract, Photosome, Vitamin E und Gemische davon.

6. Sonnenschutzspray nach einem der Ansprüche 1 bis 5, worin die weiteren Hilfsstoffe einen Komplex aus NTC-Komplex, Vitamin E, Squalane & Dunaliella Salina Extract und ein Gemisch von Isoamyl Cocoate und Phenoxyethyl Caprylate, letzteres im Verhältnis 1:1 bis 1:2, umfassen.

7. Sonnenschutzspray nach einem der Ansprüche 1 bis 6, worin der Spray SPF 30 folgende Bestandteile enthält, in Gew.-%:
| | |
|---|---|
| C12-15 Alkyl Benzoate | 20 bis 25 |
| Dicaprylyl Carbonate | 3 bis 8 |
| Ethylhexyl Methoxycinnamate | 8 bis 10 |
| Ethylhexyl Triazone | 2 bis 3 |
| Bis-Ethylhexyloxyphenol Methoxyphenyl Triazine | 4 bis 6 |
| Meadowfoam Oil | 1 bis 2 |
| Canola Oil | 1 bis 3 |
| Diisopropyl Sebacate | 3 bis 7 |
| Cyclopentasiloxane | 8 bis 12 |
| Dimethicone | 0,1 bis 1,5 |
| Ascorbyl Palmitate | 0,05 bis 0,1 |
| Propylheptyl Caprylate | 1 bis 1,5 |
| Tocopheryl Acetate | 0,1 bis 0,2 |
| NTC-Komplex | 0,01 bis 0,02 |
| Ethanol | 30 bis q.s. |
| Acrylates / Octylacrylamide Copolymer | 0,3 bis 0,5 |
| Coffea Arabica Seed Oil | 0,05 bis 0,1 |
| Phytosolve 9940 | 0,05 bis 0,15 |
| Squalane & Dunaliella Salina Extract | 0,05 bis 0,1 |
| Photosomes | 0,01 bis 0,1 |
| Isoamyl Cocoate, Phenoxyethyl Caprylate | 1 bis 2 |
| Vitamin E | 0,5 bis 1 |
| Parfümöl | 0,05 bis 0,2. |

8. Sonnenschutzspray nach einem der Ansprüche 1 bis 6, worin der Spray SPF 20 folgende Bestandteile enthält, in Gew.-%:
| | |
|---|---|
| C12-15 Alkyl Benzoate | 20 bis 25 |
| Dicaprylyl Carbonate | 3 bis 8 |
| Ethylhexyl Methoxycinnamate | 5 bis 6 |
| Ethylhexyl Triazone | 1 bis 2 |
| Bis-Ethylhexyloxyphenol Methoxyphenyl Triazine | 0,5 bis 1,5 |
| Meadowfoam Oil | 1 bis 1,5 |
| Diisopropyl Sebacate | 5 bis 5,5 |
| Ascorbyl Palmitate | 0,05 bis 0,1 |
| Propylheptyl Caprylate | 1 bis 1,5 |
| Tocopheryl Acetate | 0,1 bis 0,15 |
| NTC-Komplex | 0,01 bis 0,02 |
| Ethanol | 30 bis q.s. |
| Acrylates / Octylacrylamide Copolymer | 0,3 bis 0,5 |
| Coffea Arabica Seed Oil | 0,05 bis 0,1 |
| Phytosolve 9940 | 0,05 bis 0,9 |
| Squalane & Dunaliella Salina Extract | 0,05 bis 0,1 |
| Photosomes | 0,01 bis 0,1 |
| Isoamyl Cocoate, Phenoxyethyl Caprylate | 1 bis 2 |
| Vitamin E | 0,5 bis 1 |
| Parfümöl | 0,05 bis 0,2. |

9. Sonnenschutzspray nach einem der Ansprüche 1 - 8, worin die Brookfield-Viskosität des Sprays im Bereich von 1 - 10 mPa·s liegt.

10. Sonnenschutzspray zur Anwendung in einem Verfahren zur Behandlung der Haut, bei dem man die Haut durch Entfernung fester Partikel reinigt, einen Sonnenschutzspray gemäß Anspruch 1 aufsprüht, den Spray 5 bis 10 Minuten trocknen läßt und gegebenenfalls einen zweiten dünnen Film aufsprüht und diesen in gleicher Weise trocknen läßt.

11. Verfahren zur Behandlung der Haut mit einem Sonnenschutzspray gemäß Anspruch 1, bei dem man die Haut durch Entfernung fester Partikel reinigt, einen Sonnenschutzspray gemäß Anspruch 1 aufsprüht, den Spray 5 bis 10 Minuten trocknen läßt und gegebenenfalls einen zweiten dünnen Film aufsprüht und diesen in gleicher Weise trocknen läßt.
